# NOUVEAU FASCICULE DE BREVET EUROPEEN

(11) **EP 1 352 967 B2**
(45) Date de publication et mention de la décision concernant l'opposition: **19.11.2008**
(45) Mention de la délivrance du brevet: 31.08.2005
(21) Numéro de dépôt: 03370016.2
(22) Date de dépôt: 09.04.2003
(51) Int. Cl.: C12P 19/14

(54) **Procédé de production en continu de galacto-oligosaccharides**
Verfahren zur kontinuierlichen Herstellung von Galaktooligosacchariden
Process for continuous production of galacto-oligosaccharides

(30) Priorité: 10.04.2002 FR 0204493
(43) Date de publication de la demande: 15.10.2003
(73) Titulaire: Eurodia Industrie, 91320 Wissous (FR)
(72) Inventeur: Sawatzki, Gunther, 35516 Muenzenberg (DE)
(74) Mandataire: Hennion, Jean-Claude

(56) Documents cités:
- EP-A- 0 272 095
- EP-A- 0 663 224
- FR-A- 2 677 555
- SCHULTE M ET AL: "CONTINUOUS PREPARATIVE LIQUID CHROMATOGRAPHY IN THE DOWNSTREAM PROCESSING OF BIOTECHNOLOGICAL PRODUCTS" ACTA BIOTECHNOLOGICA, AKADEMIE VERLAG, BERLIN, DE, vol. 20, no. 1, 2000, pages 3-15, XP008010151 ISSN: 0138-4988
- BEOM K ET AL: "SIMULTANEOUSLY CONTINUOUS SEPARATION OF GLUCOSE, MALTOSE, AND MALTOTRIOSE USING A SIMULATED MOVING-BED ADSORBER" BIOSCIENCE BIOTECHNOLOGY BIOCHEMISTRY, JAPAN SOC. FOR BIOSCIENCE, BIOTECHNOLOGY AND AGROCHEM. TOKYO, JP, vol. 56, no. 5, 1992, pages 801-802, XP001042244 ISSN: 0916-8451

## Description

La présente invention concerne la production de galacto oligosaccharides en vue notamment de leur utilisation comme compléments alimentaires, notamment prébiotiques.

L'utilisation de carbohydrate comme complément alimentaire est bien connue notamment dans les ingrédients fonctionnels et plus particulièrement ceux qui sont dénommés prébiotiques du fait qu'ils développent une bio-activité prébiotique en supportant la croissance des bactéries salubres dans le gros intestin. Parmi ces carbohydrates on connaît les galacto oligosaccharides dont on a démontré qu'ils exerçaient un effet biologique (A) (T.Sako, K. Matsumoto, R. Tanaka, Recent progress on research and application of non-digestible galacto-oligosaccharides, International Diary Journal 9 (1999), 69-80).

Les galacto oligosaccharides sont produits à partir du lactose du lait grâce à une réaction enzymatique avec la β-galactosidaze.

On a déjà proposé notamment par le document EP.O.272.095 la production d'une solution contenant des galacto oligosaccharides répondant à la formule Gal-(Gal)ₙ-Glu, dans laquelle Gal représente un résidu de galactose, Glu représente un résidu de glucose et n représente un nombre entier de 1 à 4. Le procédé de préparation décrit dans ce document consiste à procéder à une réaction enzymatique du lactose avec un enzyme ou un micro-organisme apte à réaliser la transformation dudit lactose en galacto oligosaccharide puis à séparer les composants contenus dans la solution réactionnelle par chromatographie en utilisant une résine échangeuse cationique acide fort et à collecter les fractions contenant une concentration élevée de galacto oligosaccharide à partir des éluats de la colonne de chromatographie, l'enzyme en question est par exemple du type β-galactosidaze. Dans un mode de réalisation, la résine échangeuse cationique acide fort est à base d'un copolymère styrène divinyl benzène et possède des groupes échangeurs d'ions du type sulfonique, sous la forme Na+.

Il est de l'avis du demandeur que le procédé donné par le document précité EP.0272095 n'est pas totalement satisfaisant, notamment ne permettant pas un fonctionnement continu dans de bonnes conditions.

Le procédé de invention est un procédé continu de production de galactooligosaccharides à partir de lactose.
Ce procédé consiste :
a) à remplir un réacteur avec un bain contenant une solution de lactose et des enzymes du type β-galactosidase, en vue de produire par réaction enzymatique des monosaccharides (glucose Glu et galactose Gal) et des oligosaccharides parmi lesquels des galactooligosaccharides,
b) à alimenter en continu le réacteur avec un débit donné de lactose frais,
c) à faire circuler en continu le bain provenant du réacteur, avec un débit donné, dans un dispositif membranaire d'ultra filtration, réalisant la séparation d'une part d'un perméat contenant un reste de lactose, les monosaccharides et les oligosaccharides et d'autre part du bain résiduel contenant les enzymes et la majorité du lactose,
d) à transférer en continu le bain résiduel dans le réacteur,
e) à faire passer en continu le perméat sur un ou plusieurs lits mobiles simulés (SMB) garnis d'une résine échangeuse d'ions cationiques forts, réalisant la séparation de trois fractions :
   - une première fraction contenant principalement du lactose,
   - une seconde fraction contenant principalement les galactooligosaccharides,
   - et une troisième fraction contenant principalement les monosaccharides,
f) à transférer en continu la première fraction dans le réacteur,
g) à collecter indépendamment l'un de l'autre les seconde et troisième fractions.

Ainsi de manière caractéristique selon la présente invention, sont combinées, dans un processus continu, une séparation membranaire de type ultra-filtration et une séparation SMB, cette dernière étant plus efficace du fait de l'élimination du recyclage dans le bain de réaction enzymatique des enzymes et d'une grande part du lactose n'ayant pas encore réagi. Il est ainsi possible d'obtenir une troisième fraction particulièrement riche en galactooligosaccharides et un rendement très élevé de transformation du lactose.

Les galactooligosaccharides obtenus dans la troisième fraction ont pour formule Gal-(Gal)ₙ-Glu dans laquelle Gal est un motif de galactose, Glu un motif de glucose et n est de 1 à 5.

Le débit de lactose frais dans le réacteur est déterminé, en fonction du débit des trois fractions sortant du SMB, pour maintenir constant le volume du bain de réaction contenu dans le réacteur.

La concentration en lactose dans le bain de réaction est maintenue constamment entre 8 et 60%, de préférence entre 20 et 60%.

Les conditions de la réaction enzymatique (pH, température, quantité d'enzymes) sont déterminées en sorte d'obtenir un taux de conversion du lactose en monosaccharides inférieur à 30% et un taux de conversion en oligosaccharides supérieur à 4%. Par exemple le pH est compris entre 3 et 8, la température est supérieure ou égale à 25°C et la concentration en enzyme est entre 1 et 200 unités/ml.

Pour obtenir le taux de conversion du lactose en monosaccharides le plus bas possible, on ajuste le débit de circulation du bain réactionnel dans le dispositif membranaire d'ultrafiltration (ce qui conditionne le débit du perméat) en sorte que le temps moyen de séjour du bain réactionnel dans le réacteur est compris entre 0,5 et 4 heures.

La seconde fraction obtenue selon le procédé de l'invention a un taux de pureté élevé en galactooligosaccharides, supérieur à 90%, contenant moins de 5% de lactose et moins de 5% de monosaccharides.

Dans les conditions optimales, vues ci-dessus, on a obtenu une seconde fraction contenant 94% de galactooligosaccharides, 2,5% en lactose et 1% de glucose.

Du fait du faible taux de monosaccharide, il est possible de transformer cette seconde fonction en poudre par des techniques connues, notamment séchage/congélation ou séchage/pulvérisation.

De plus, avec cette pureté en galactooligosaccharides, et un faible taux de lactose et de monosaccharides, cette seconde fraction - poudre ou solution - peut être utilisée comme prébiotique, en particulier pour des personnes qui ont une intolérance au lactose.

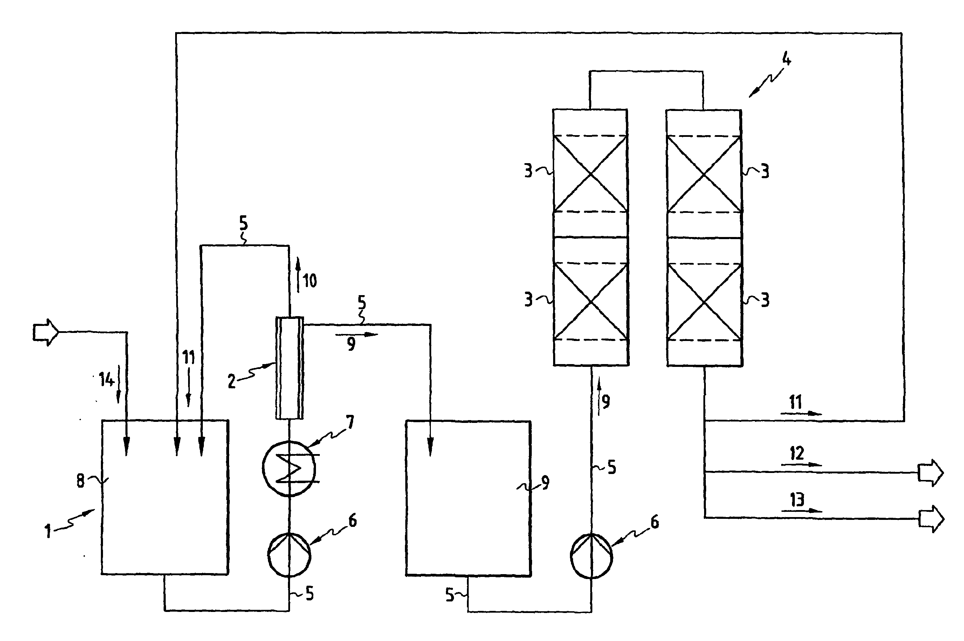

La présente invention sera mieux comprise à la lecture de la description qui va être faite d'un exemple préféré de réalisation de production, en continu, d'une fraction particulièrement riche en galactooligosaccharides, utilisable comme prébiotique, illustré par le dessin annexé dans lequel la figure unique est une représentation, sous forme de diagramme, de l'installation permettant la mise en oeuvre du procédé.

Cette installation comporte un réacteur 1, un dispositif membranaire d'ultrafiltration 2 et quatre colonnes 3, montées en série et formant un ensemble SMB 4. Elle comporte également des tuyauteries 5 et pompes de circulation 6 nécessaires au transfert des liquides. Plus particulièrement, sur la tuyauterie 5 transférant le bain contenu dans le réacteur 1 jusqu'au dispositif membranaire 2 d'ultrafiltration, se trouve une pompe 6 puis un échangeur de chaleur 7, permettant de réguler la température du bain 8 réactionnel.

Ce bain 8 réactionnel est composé initialement, lors du remplissage du réacteur 1, avec une solution de lactose à laquelle a été ajoutée une certaine quantité d'enzymes permettant, par réaction enzymatique, de transformer le lactose en monosaccharides et en oligosaccharides, parmi lesquels les galactooligosaccharides que l'on cherche à concentrer. L'enzyme en question est notamment la β-galactosidase provenant de Kluyveromyces lactis or Aspergillus oryzae. Le dispositif membranaire d'ultrafiltration 2 est de préférence un module à fibres creuses, avec un pouvoir d'arrêt, en masse moléculaire, de l'ordre de 10.000. Le bain réactionnel 8 passe dans le circuit interne du module et le bain résiduel 10 et retourne dans le réacteur 1, tandis que le perméat 9 sortant du module de fibres creuses est envoyé dans l'ensemble SMB 4.

On comprend que la réaction enzymatique se produit dans tout le bain réactionnel 8 qui se trouve non seulement dans le réacteur 1 mais également dans les tuyauteries 5 et dans le dispositif membranaire d'ultrafiltration 2.

La fonction de ce dispositif membranaire 2 est d'extraire du bain réactionnel 8 les monosaccharides et les oligosaccharides qui se sont formés. En réalité le dispositif membranaire 2 ne permet pas l'arrêt total de tout le lactose, une partie dudit lactose se retrouvant dans le perméat 9 avec les monosaccharides et les oligosaccharides. Le bain résiduel 10 sortant du dispositif membranaire 2, comprenant les enzymes et le lactose résiduel, est renvoyé dans le réacteur 1.

Le perméat 9 est envoyé grâce à une pompe 6 dans l'ensemble SMB (Simulated Moving Bed) qui comporte quatre colonnes, remplies de billes de résine d'un échangeur d'ions fort sous une forme cationique, par exemple connue sous la référence DIAION UBK 535K, Resindion S.R.L., Mitsubishi Chemical Corporation, Binasco, Italy. Cet ensemble fonctionne dans le mode ISMB (Improved SMB mode), faisant l'objet des documents EP.10767 et EP.663224. Cette technique est améliorée du fait qu'elle permet d'obtenir des concentrations plus élevées tout en évitant de recirculer le perméat durant le fractionnement. De plus elle permet de travailler avec une charge volumétrique plus importante, un volume plus réduit de résine par rapport à la technique classique SMB, une consommation d'eau et d'énergie plus basse.

En sortie de l'ensemble SMB 4, on recueille trois fractions concentrées, une première fraction 11, chargée en lactose, une seconde fraction 12 chargée en galactooligosaccharides et une troisième fraction 13 chargée en monosaccharides. C'est bien sûr la seconde fraction 12 qui est recherchée dans le cadre du procédé de la présente invention.

La première fraction 11, chargée en lactose, est renvoyée dans le réacteur 1 en sorte d'être recyclé dans le bain réactionnel 8.

De plus pour un fonctionnement continu de l'installation, le réacteur 1 est alimenté de manière continue ou éventuellement séquencée par du lactose frais 14 en sorte de maintenir le volume du bain réactionnel 8 dans le réacteur 1 sensiblement constant, ainsi que les conditions opératoires suivantes : concentration en lactose comprise entre 10 et 60%, concentration en enzymes de 1 à 200 unités/ml, pH de 3 à 8, température supérieure à 25°C, de préférence de l'ordre de 50°C.

Le débit de circulation du bain réactionnel 8 dans le dispositif membranaire 2 d'ultrafiltration est ajusté en sorte que le temps moyen de séjour du bain réactionnel 8 dans le réacteur soit compris entre ½ h et 4 h. Cette disposition particulière a pour but de limiter, au cours de la réaction enzymatique, la transformation du lactose en monosaccharides, du type glucose et galactose. Les conditions opératoires ci-dessus permettent d'obtenir une conversion enzymatique continue donnant de l'ordre de 4 à 35% d'oligosaccharides et un taux de conversion en monosaccharides inférieur à 30%.

La résine qui est mise en oeuvre, de manière préférée, dans l'ensemble SMB 4 est composée d'un copolymère styrène divinyl benzène qui comporte des groupements échangeurs d'ions acide fort, de type sulfonique et qui est sous la forme Ca2+. La séparation des différents composants dans l'ensemble SMB est basée sur la taille moléculaire.

En mettant en oeuvre le procédé de l'invention selon l'ensemble des fonctionnements et des conditions décrits ci-dessus, en particulier s'agissant de l'ensemble SMB en mettant en oeuvre les conditions décrites dans le document EP.663224, il a été possible d'obtenir une seconde fraction 12 ayant une concentration en galactooligosaccharides supérieure à 90% et contenant moins de 5% de lactose et moins de 5% de monosaccharides.

Dans un exemple précis de réalisation, on a traité en continu 200l d'une solution contenant 40% en poids de lactose. Le volume contenu dans le réacteur 1 a été maintenu à environ 10l. L'échangeur de chaleur 7 a été réglé pour maintenir la température du bain réactionnel 8 à environ 50°C. Le dispositif membranaire d'ultrafiltration 2 consistait en un module de fibres creuses du type ROMICON PM 10, avec une surface active de 0,5m². Le bain réactionnel a été incubé avec 0,5% en volume d'une solution enzymatique (Maxilact 2000 L), ce qui équivaut à environ 100 unités/ml. Le débit de bain réactionnel à travers le module de fibres creuses a été ajusté à 2,75 l/h. Le perméat 9 obtenu a été alimenté dans l'ensemble SBM 4, comportant quatre colonnes 3 remplies avec des billes de résine DIAION UBK 535 K. Le diamètre intérieur des colonnes était de 2,75cm et leur longueur de 50 cm. Cet ensemble a été porté à une température de l'ordre de 50°C. Les paramètres relatifs au fonctionnement de l'ensemble ISBM ont été calculés après une simulation en ordinateur, conformément au tableau 7 du document EP.663224.

La seconde fraction 12 collectée, en sortie de l'ensemble SBM 4, contenait 94% de galactooligosaccharides, 2,5% de lactose et 1% de glucose.

La pureté en galactooligosaccharides obtenue grâce au procédé de l'invention, alliée aux faibles quantités de monosaccharides et de lactose, permet l'utilisation de cette fraction comme prébiotique tout particulièrement pour les personnes qui présentent une intolérance au lactose.

De plus du fait de la faible teneur en monosaccharides, il est possible de transformer cette fraction en poudre par toute technique connue, notamment par séchage/congélation ou par séchage/pulvérisation.

Le fait que le procédé de l'invention soit réalisé de manière continue, avec une très faible quantité d'eau utilisée pour la séparation, permet de garantir un haut débit, ce qui réduit d'autant le coût de production.

Le recyclage du lactose n'ayant pas réagi, tant dans le bain résiduel 10 sortant du dispositif membranaire d'ultrafiltration 2 que de la première fraction 11 sortant de l'ensemble SMB4, permet d'obtenir un rendement en galactooligosaccharides non atteint par les techniques connues.

## Revendications

1. Procédé continu de production d'une fraction concentrée (12) de galactooligosaccharides à partir de lactose, consistant :
a) à remplir un réacteur (1) avec un bain contenant une solution de lactose et des enzymes du type β-galactosidase, en vue de produire par réaction enzymatique des monosaccharides (glucose Glu et galactose Gal) et des oligosaccharides parmi lesquels des galactooligosaccharides,
b) à alimenter en contenu le réacteur (1) avec un débit donné de lactose frais (14),
c) à faire circuler en continu le bain réactionnel (8), avec un débit donné, dans un dispositif membranaire d'ultra filtration (2), réalisant la séparation d'une part d'un perméat (9) contenant un reste de lactose, les monosaccharides et les oligosaccharides et d'autre part du bain résiduel (10) contenant les enzymes et le lactose résiduel,
d) à transférer en continu le bain résiduel (10) dans le réacteur (1),
e) à faire passer en continu le perméat (9) sur un ou plusieurs lits mobiles simulés (SMB) (4) garnis d'une résine échangeuse d'ions cationiques forts, réalisant la séparation de trois fractions :
- une première fraction (11) contenant principalement du lactose,
- une seconde fraction (12) contentant principalement les galactooligosaccharides,
- et une troisième fraction (13) contenant principalement les monosaccharides,
f) à transférer en continu la première fraction (11) dans le réacteur (1),
g) à collecter indépendamment l'un de l'autre les seconde (12) et troisième (13) fractions.

2. Procédé selon la revendication 1 **caractérisé en ce que** le débit de lactose frais (14) est déterminé en fonction du débit des trois fractions sortant du SMB, pour maintenir sensiblement constant le volume du bain réactionnel: (8).

3. Procédé selon l'une des revendications 1 ou 2 **caractérisé en ce que** la concentration en lactose dans le bain réactionnel (8) est maintenue constamment entre 8 et 60%, de préférence entre 20 et 60%.

4. Procédé selon la revendication 3 **caractérisé en ce que** les conditions de la réaction enzymatique (pH, température, quantité d'enzymes) dans le bain réactionnel (8) sont déterminées en sorte d'obtenir un taux de conversion du lactose en monosaccharides inférieur à 30% et un taux de conversion en oligosaccharides supérieur à 4%.

5. Procédé selon la revendication 4 **caractérisé en ce que** le pH est compris entre 3 et 8, la température est supérieurs ou égale à 25°C et la concentration en enzyme est entre 1 et 200 unité/ml.

6. Procédé selon l'une des revendications 1 à 5 **caractérisé en ce qu'**on ajuste le débit de circulation du bain réactionnel (8) dans le dispositif membranaire d'ultrafiltration (2) en sorte que le temps moyen de séjour du bain réactionnel (8) dans le réacteur (1) est compris entre 0,5 et 4 heures.

## Claims

1. Process for continuous production of a concentrated fraction (12) of galacto-oligosaccharides from lactose, consisting of:
a) filling a reactor (1) with a bath containing a solution of lactose and β-type galactosidase enzymes for the production by enzymatic reaction of monosaccharides (glucose Glu and galactose Gal) and oligosaccharides among which galactooligosaccharides,
b) continuously supplying the reactor (1) with a given flow of fresh lactose (14),
c) causing the reaction bath (8) to circulate continuously, at a given flow rate, in an ultra-filtration membrane device (2), to achieve the separation firstly of a permeate (9) containing a remainder of lactose, the monosaccharides and oligosaccharides and secondly of the residual bath (10) containing the enzymes and residual lactose,
d) continuously transferring the residual bath (10) into the reactor (1),
e) continuously causing the permeate (9) to pass through one or more simulated mobile beds (SMB) (4) packed with a strong cation-exchange resin, to achieve the separation of three fractions:
- a first fraction (11) chiefly containing lactose,
- a second fraction (12) chiefly containing the galacto-oligosaccharides,
- a third fraction (13) chiefly containing the monosaccharides,
f) continuously transferring the first fraction (11) into the reactor (1),
g) collecting independently from each other the second (12) and third (13) fractions.

2. Process as in claim 1, **characterized in that** the flow of fresh lactose (14) is determined in relation to the flow of the three fractions leaving the SMB to as to maintain the volume of reaction bath (8) substantially constant.

3. Process as in either of claims 1 or 2, **characterized in that** the lactose concentration in the reaction bath (8) is constantly maintained between 8 and 60%, preferably between 20 and 60%.

4. Process as in claim 3, **characterized in that** the conditions of the enzymatic reaction (pH, temperature, quantity of enzymes) in the reaction bath (8) are determined so as to obtain a conversion rate of lactose into monosaccharides of less than 30% and a conversion rate into oligosaccharides of more than 4%.

5. Process as in claim 4, **characterized in that** the pH lies between 3 and 8, the temperature is 25°C or higher and the enzyme concentration is between 1 and 200 units/ml.

6. Process as in any of claims 1 to 5, **characterized in that** the circulation flow rate of the reaction bath (8) in the ultra-filtration membrane device (2) is adjusted so that the mean residence time of the reaction bath (8) in the reactor (1) lies between 0.5 and 4 hours.

## Patentansprüche

1. Kontinuierliches Verfahren zur Herstellung einer konzentrierten Fraktion (12) von Galactooligosacchariden aus Lactose, darin bestehend:
a) einen Reaktor (1) mit einem Bad zu befüllen, das eine Lösung aus Lactose und Enzymen des Typs β-Galactosidase enthält, um durch Enzymreaktion Monosaccharide (Glucose Glu und Galactose Gal) und Oligosaccharide, unter anderem Galactooligosaccharide, zu erzeugen,
b) den Reaktor (1) kontinuierlich mit einer gegebenen Menge an frischer Lactose (14) zu speisen,
c) das Reaktionsbad (8) kontinuierlich mit einer gegebenen Menge in einer Membranvorrichtung (2) zur Ultrafiltration zirkulieren zu lassen, die die Trennung in einerseits ein Permeat (9), das einen Lactoserest, die Monosaccharide und die Oligosaccharide enthält, und andererseits das Restbad (10), das die Enzyme und die Restlactose enthält, durchführt,
d) kontinuierlich das Restbad (10) in den Reaktor (1) weiterzuleiten,
e) kontinuierlich das Permeat (9) auf ein oder mehrere simulierte mobile Betten (SMB) (4) überzuleiten, die mit einem lonenaustauschharz für starke Kationen versehen ist, das die Trennung in drei Fraktionen durchführt:
- eine erste Fraktion (11), die hauptsächlich Lactose enthält,
- eine zweite Fraktion (12), die hauptsächlich die Galactooligosaccharide enthält,
- eine dritte Fraktion (13), die hauptsächlich die Monosaccharide enthält,
f) kontinuierlich die erste Fraktion (11) in den Reaktor (1) weiterzuleiten,
g) unabhängig voneinander die erste (12) und dritte (13) Fraktion zu sammeln.

2. Verfahren nach Anspruch 1, **dadurch gekennzeichnet, daß** die Menge an frischer Lactose (14) in Abhängigkeit von der Menge der drei aus dem SMB austretenden Fraktionen bestimmt wird, um das Volumen des Reaktionsbades (8) im wesentlichen konstant zu halten.

3. Verfahren nach einem der Ansprüche 1 oder 2, **dadurch gekennzeichnet, daß** die Lactosekonzentration in dem Reaktionsbad (8) konstant zwischen 8 und 60 %, vorzugsweise zwischen 20 und 60 %, gehalten wird.

4. Verfahren nach Anspruch 3, **dadurch gekennzeichnet, daß** die Bedingungen der Enzymreaktion (pH-Wert, Temperatur, Enzymmenge) in dem Reaktionsbad (8) derart bestimmt werden, daß eine Umwandlungsrate von Lactose in Monosaccharide von unter 30 % und eine Umwandlungsrate in Oligosaccharide von über 4 % erzielt wird.

5. Verfahren nach Anspruch 4, **dadurch gekennzeichnet, daß** der pH-Wert zwischen 3 und 8 liegt, die Temperatur größer oder gleich 25 °C ist und die Enzymkonzentration zwischen 1 und 200 Einheiten/ml beträgt.

6. Verfahren nach einem der Ansprüche 1 bis 5, **dadurch gekennzeichnet, daß** die Zirkulationsmenge des Reaktionsbades (8) in der Membranvorrichtung (2) zur Ultrafiltration derart eingestellt wird, daß die durchschnittliche Aufenthaltszeit des Reaktionsbades (8) im Reaktor (1) zwischen 0,5 und 4 Stunden beträgt.
